# EUROPEAN PATENT APPLICATION

(11) **EP 4 843 558 A1**
(43) Date of publication of application: **25.11.2026**
(21) Application number: 26178392.2
(22) Date of filing: 13.05.2026
(51) Int. Cl.: A61K 8/67, A61K 8/34, A61Q 19/00, A61Q 19/08

(54) **COMPOSITION PROMOTING SKIN ABSORPTION OF RIBOFLAVIN 5'-PHOSPHATE**

(30) Priority: 20.05.2025 KR 20250065313; 20.10.2025 KR 20250151646
(71) Applicant: Amorepacific Corporation, Seoul 04386 (KR)
(72) Inventor: CHOI, Joonho, Yongin-si Gyeonggi-do 17074 (KR); KIM, Jihoon, Yongin-si, Gyeonggi-do 17074 (KR); LEE, Somi, Yongin-si Gyeonggi-do 17074 (KR); KIM, Jihyun, Yongin-si, Gyeonggi-do 17074 (KR); LEE, Seungmin, Yongin-si, Gyeonggi-do 17074 (KR); LEE, Eun Soo, Gyeonggi-do 17074 (KR); HONG, Sohyun, Yongin-si, Gyeonggi-do 17074 (KR)
(74) Representative: Germain Maureau

(57) **Abstract**

Disclosed in the present specification is a composition for promoting skin absorption of riboflavin 5'-phosphate. According to the present disclosure, by providing riboflavin 5'-phosphate or a salt thereof as a formulation dissolved in a solvent comprising pentylene glycol, an amount of skin absorption of the riboflavin 5'-phosphate or the salt thereof is promoted to allow the riboflavin 5'-phosphate or the salt thereof to be absorbed into the skin in a short time within 5 minutes after application, and accordingly, an excellent effect of improving skin wrinkles or elasticity may be provided.

## Description

### BACKGROUND OF THE INVENTION

### Field of the Invention

Disclosed in the present specification is a composition for promoting skin absorption of riboflavin 5'-phosphate.

### Description of the Related Art

Riboflavin 5'-phosphate has been known to be effective in improving skin wrinkles and enhancing skin elasticity through efficacies such as antioxidation, collagen expression, and inhibition of skin fibroblast elastase (SFE) and MMP-1, and thus has been continuously utilized as an active ingredient in the field of cosmetics.

However, the riboflavin 5'-phosphate has a limitation in that skin permeability is significantly low due to a property of high hydrophilicity. Due to this, even if the riboflavin 5'-phosphate is included in a cosmetic composition, effective delivery into the skin is difficult, so there is a problem in that manifestation of efficacy in the actual skin is insignificant.

Therefore, in order to sufficiently utilize excellent skin improvement efficacy of the riboflavin 5'-phosphate, a technical solution capable of improving skin permeability is required.

### SUMMARY OF THE INVENTION

The present disclosure is directed to providing a composition for promoting skin absorption of riboflavin 5'-phosphate.

To solve the above problem, an embodiment of the present disclosure provides a composition of a skin application formulation, wherein the composition comprises riboflavin 5'-phosphate or a salt thereof as an active ingredient, the formulation is a form in which the riboflavin 5'-phosphate or the salt thereof is dissolved in a solvent comprising pentylene glycol, and in the formulation, a degree of skin absorption of the riboflavin 5'-phosphate or the salt thereof is enhanced.

In compositions according to embodiments of the present disclosure, skin absorption of riboflavin 5'-phosphate or a salt thereof is promoted so that it may be absorbed into the skin in a short time within 5 minutes after application, and skin absorption depth may also be increased. Accordingly, the compositions according to the embodiments of the present disclosure may effectively provide useful efficacies for the skin of the riboflavin 5'-phosphate or the salt thereof, such as an effect of improving skin wrinkles or elasticity.

### BRIEF DESCRIPTION OF THE DRAWINGS

FIG. 1 is a view in which an absorption amount into the skin of riboflavin 5'-phosphate according to a type of an absorption promoter is measured in Test Example 1 of the present disclosure, and the measured absorption amount is illustrated as a ratio (times) relative to Comparative Example 1 not comprising an absorption promoter.
FIG. 2 is a view illustrating comparison of results of measuring an absorption depth into the skin of riboflavin 5'-phosphate according to a type of an absorption promoter in Test Example 2 of the present disclosure.
FIG. 3 is a view in which an absorption amount into the skin of riboflavin 5'-phosphate according to a content of pentylene glycol used as an absorption promoter is measured in Test Example 3 of the present disclosure, and the measured absorption amount is illustrated as a ratio (times) relative to Comparative Example 1 not comprising an absorption promoter.
FIG. 4 is a view illustrating a result of comparing an absorption promoting effect of pentylene glycol on riboflavin 5'-phosphate with an absorption promoting effect on tocopherol, according to Test Example 4 of the present disclosure.

### DETAILED DESCRIPTION OF THE INVENTION

Hereinafter, exemplary embodiments of the present disclosure will be described in detail with reference to the accompanying drawings.

The embodiments of the present disclosure disclosed herein are exemplified for the purpose of description only, and the embodiments of the present disclosure may be implemented in various forms. Since the present disclosure may be subject to various modifications and take various forms, it should be understood to include all modifications, equivalents, or substitutes included in the technical teachings and scope of the present disclosure. Singular expressions include plural expressions unless clearly described as different meanings in the context. In the present application, the terms "comprises," "comprising," "includes," "including," "containing," "has," "having" or other variations thereof are inclusive and therefore specify the presence of stated features, integers, steps, operations, constituent elements, components (or parts), and/or combinations thereof, but do not exclude the presence or addition of one or more other features, integers, steps, operations, constituent elements, components (or parts), and/or combinations thereof.

An embodiment of the present disclosure provides a composition of a skin application formulation, wherein the composition comprises riboflavin 5'-phosphate or a salt thereof as an active ingredient, the formulation is a form in which the riboflavin 5'-phosphate or the salt thereof is dissolved in a solvent comprising pentylene glycol, and in the formulation, a degree of skin absorption of the riboflavin 5'-phosphate or the salt thereof is enhanced.

In the present disclosure, the riboflavin 5'-phosphate is an active form of B2 and is mainly converted into flavin protein in a body to act as a cofactor of various enzymes, and when used on skin, may provide various useful efficacies to the skin by antioxidation action. However, when the riboflavin 5'-phosphate or the salt thereof is applied to the skin, there was a limitation in that manifestation of efficacy in actual skin is insignificant due to low permeability into the skin due to hydrophilicity.

In one aspect above, an embodiment of the present disclosure comprises pentylene glycol in a solvent as an absorption promoter for promoting skin absorption of the riboflavin 5'-phosphate or the salt thereof, so that the riboflavin 5'-phosphate or the salt thereof, which is an active ingredient, may be rapidly absorbed into the skin in a short time within 5 minutes, and thus useful efficacies for the skin of the riboflavin 5'-phosphate or the salt thereof may be substantially manifested in the skin.

In an embodiment, the composition may comprise an aqueous solution in which the riboflavin 5'-phosphate or the salt thereof is dissolved in the pentylene glycol.

In an embodiment, the salt of riboflavin 5'-phosphate is not limited as long as it is a cosmetic composition or a pharmaceutically acceptable salt, and for example, may comprise base addition salts and amino acid salts. For example, the salt of the riboflavin 5'-phosphate may comprise inorganic base salts such as sodium salt, potassium salt, calcium salt, magnesium salt, copper salt, zinc salt, aluminum salt, and ammonium salt; organic base salts such as triethylammonium salt, triethanolammonium salt, pyridinium salt, and diisopropylammonium salt; and amino acid salts such as lysine salt, arginine salt, histidine salt, aspartate, and glutamate. In an embodiment, the salt of the riboflavin 5'-phosphate may be a water-soluble salt, and specifically, may be an inorganic base salt. More specifically, the salt of the riboflavin 5'-phosphate may be an alkali metal salt such as a sodium salt or a potassium salt of riboflavin 5'-phosphate.

In an embodiment, the composition may comprise the riboflavin 5'-phosphate or the salt thereof in an amount of 0.0001 to 10 wt% based on a total weight of the composition. Specifically, a content of the riboflavin 5'-phosphate or the salt thereof may be 0.0001 wt% or more, 0.001 wt% or more, 0.01 wt% or more, 0.05 wt% or more, 0.1 wt% or more, 0.5 wt% or more, 1.0 wt% or more, 1.5 wt% or more, 2 wt% or more, 2.5 wt% or more, 3 wt% or more, 3.5 wt% or more, 4 wt% or more, 4.5 wt% or more, 5 wt% or more, 5.5 wt% or more, 6 wt% or more, 6.5 wt% or more, 7 wt% or more, 7.5 wt% or more, 8 wt% or more, 8.5 wt% or more, 9 wt% or more, or 9.5 wt% or more, and may be 10 wt% or less, 9.5 wt% or less, 9 wt% or less, 8.5 wt% or less, 8 wt% or less, 7.5 wt% or less, 7 wt% or less, 6.5 wt% or less, 6 wt% or less, 5.5 wt% or less, 5 wt% or less, 4.5 wt% or less, 4 wt% or less, 3.5 wt% or less, 3 wt% or less, 2.5 wt% or less, 2 wt% or less, 1.5 wt% or less, 1 wt% or less, 0.5 wt% or less, 0.1 wt% or less, 0.05 wt% or less, 0.01 wt% or less, or 0.001 wt% or less, based on the total weight of the composition. When the content is less than 0.0001 wt%, a desired useful effect may appear insignificantly, and when it exceeds 10 wt%, solubility in a formulation may decrease.

In an embodiment, the pentylene glycol may be comprised in an amount of 1 to 40 wt% based on the total weight of the composition. Specifically, the pentylene glycol may be comprised in an amount of 1 wt% or more, 5 wt% or more, 10 wt% or more, 15 wt% or more, 20 wt% or more, 25 wt% or more, 30 wt% or more, or 35 wt% or more, and may be comprised in an amount of 40 wt% or less, 35 wt% or less, 30 wt% or less, 25 wt% or less, 20 wt% or less, 15 wt% or less, 10 wt% or less, or 5 wt% or less, based on the total weight of the composition. When the pentylene glycol is comprised in an amount of less than 1 wt%, a skin absorption promoting effect of riboflavin 5'-phosphate or a salt thereof may not be significant, and when it exceeds 40 wt%, a possibility that stuffiness and stickiness are induced in terms of a sense of use may appear.

In an embodiment, a weight ratio of the riboflavin 5'-phosphate or the salt thereof to pentylene glycol may be 1: 1 to 10,000. Specifically, the weight ratio of the riboflavin 5'-phosphate or the salt thereof to pentylene glycol may be 1: 1 or more, 10 or more, 50 or more, 100 or more, 500 or more, 1,000 or more, or 5,000 or more, and may be 10,000 or less, 5,000 or less, 1,000 or less, 500 or less, 100 or less, 50 or less, or 10 or less. When pentylene glycol is comprised in an excessively small amount relative to riboflavin 5'-phosphate or a salt thereof out of the above range, a skin absorption rate of the riboflavin 5'-phosphate or the salt thereof decreases, so that the effect may not be significant when applied to the skin. When pentylene glycol is comprised in an excessive amount relative to riboflavin 5'-phosphate or a salt thereof, a content of the riboflavin 5'-phosphate or the salt thereof relatively decreases, so that a skin improvement effect of the riboflavin 5'-phosphate or the salt thereof may also decrease.

In an embodiment, the composition may be for preventing skin wrinkle generation or improving wrinkles, or preventing skin elasticity decrease or improving skin elasticity. In another embodiment, provided may be a method for preventing skin wrinkle generation or improving wrinkles, or preventing skin elasticity decrease or improving skin elasticity, comprising applying an effective amount of the riboflavin 5'-phosphate or the salt thereof and the pentylene glycol to skin of a subject in need thereof. Another embodiment may provide a use of the riboflavin 5'-phosphate or the salt thereof and pentylene glycol as an active ingredient for manufacturing a composition for preventing skin wrinkle generation or improving wrinkles, or preventing skin elasticity decrease or improving skin elasticity. Another embodiment may provide the riboflavin 5'-phosphate or the salt thereof and pentylene glycol for use in a composition for preventing skin wrinkle generation or improving wrinkles, or preventing skin elasticity decrease or improving skin elasticity.

In an embodiment, a use of the composition may be one or more of skin antioxidation, skin regeneration, or prevention or improvement of skin inflammation.

In another embodiment of the present disclosure, provided may be a method for one or more of skin antioxidation, skin regeneration, or prevention or improvement of skin inflammation, comprising applying an effective amount of the riboflavin 5'-phosphate or the salt thereof and the pentylene glycol to skin of a subject in need thereof. Another embodiment may provide a use of the riboflavin 5'-phosphate or the salt thereof and pentylene glycol for manufacturing a composition for one or more of skin antioxidation, skin regeneration, or prevention or improvement of skin inflammation. Another embodiment may provide the riboflavin 5'-phosphate or the salt thereof and pentylene glycol for use in a composition for one or more of skin antioxidation, skin regeneration, or prevention or improvement of skin inflammation.

In the embodiment above, the composition may be a cosmetic composition.

In an embodiment, an external appearance of the composition may contain a cosmetically or dermatologically acceptable medium or base. For example, it may be provided in a form of a solution, a gel, a solid, a paste anhydrous product, an emulsion obtained by dispersing an oil phase in an aqueous phase or obtained by dispersing an aqueous phase in an oil phase, a suspension, a microemulsion, a microcapsule, a microgranule, or an ionic type (liposome), a non-ionic type vesicle dispersant, a balm, a patch, a pad, a film, a mask, or the like. These compositions may be manufactured according to a conventional method in the relevant field.

In an embodiment, the cosmetic composition may be formulated into cosmetics such as an ampoule, a cream, a softening cosmetic water, an astringent cosmetic water, a nourishing cosmetic water, a nourishing cream, a gel cream, a massage cream, an essence, an eye cream, an eye essence, a cleansing cream, a cleansing foam, a cleansing water, a pack (or facial mask), a body lotion, a body cream, a body oil, and a body essence. When a formulation according to an embodiment of the present disclosure is a paste, a cream, or a gel, animal fiber, vegetable fiber, wax, paraffin, starch, tragacanth, cellulose derivatives, polyethylene glycol, silicone, bentonite, silica, talc, or zinc oxide, etc. may be used as a carrier component. When a formulation according to an embodiment of the present disclosure is a spray, lactose, talc, silica, aluminum hydroxide, calcium silicate, or polyamide powder may be used as a carrier component, and additionally, a propellant such as chlorofluorohydrocarbon, propane/butane, or dimethyl ether may be comprised. When a formulation according to an embodiment of the present disclosure is a solution or an emulsion, a solvent, a solvating agent, or an emulsifying agent is used as a carrier component, and there are, for example, water, ethanol, isopropanol, ethyl carbonate, ethyl acetate, benzyl alcohol, benzyl benzoate, propylene glycol, 1,3-butyl glycol oil, glycerol aliphatic ester, polyethylene glycol, or fatty acid ester of sorbitan. When a formulation according to an embodiment of the present disclosure is a suspension, a liquid diluent such as water, ethanol, or propylene glycol, a suspending agent such as ethoxylated isostearyl alcohol, polyoxyethylene sorbitol ester, and polyoxyethylene sorbitan ester, microcrystalline cellulose, aluminum metahydroxide, bentonite, agar, or tragacanth, etc. may be used as a carrier component. When a formulation according to an embodiment of the present disclosure is a surfactant-containing cleansing formulation, aliphatic alcohol sulfate, aliphatic alcohol ether sulfate, sulfosuccinic acid monoester, isethionate, imidazolinium derivatives, methyl taurate, sarcosinate, fatty acid amide ether sulfate, alkylamido betaine, aliphatic alcohol, fatty acid glyceride, fatty acid diethanolamide, vegetable oil, lanolin derivatives, or ethoxylated glycerol fatty acid ester, etc. may be used as a carrier component.

In the cosmetic composition according to an embodiment of the present disclosure, functional additives and components included in general cosmetic compositions may be additionally included. The functional additives may include a component selected from the group consisting of water-soluble vitamins, oil-soluble vitamins, polymer peptides, polymer polysaccharides, sphingolipids, and seaweed extract. As blending components included other than these, oil and fat components, a moisturizer, an emollient, a neutralizing agent, a surfactant, organic and inorganic pigments, organic powder, an ultraviolet absorber, an antiseptic, a germicide, an antioxidant, plant extracts, a pH adjuster, alcohol, a colorant, a fragrance, a blood circulation promoter, a cooling agent, an antiperspirant, purified water, etc. may be mentioned.

As an embodiment, the composition may be applied directly with a hand or using various application means such as a spatula, a brush, and a puff.

In an embodiment, the composition may be a pharmaceutical composition. When a composition according to an embodiment of the present disclosure is applied to a medicine, it may be formulated as a parenteral administration agent in a semi-solid or liquid form by adding an inorganic or organic carrier commonly used for an active ingredient used in an embodiment of the present disclosure. As an embodiment, the pharmaceutical composition may be a parenteral administration agent, more specifically, an agent for external application to skin. In an embodiment, the formulation and additional components of the agent for external application to skin may be equally applied to those for the cosmetic composition described in detail in the present disclosure. For example, the agent for external application to skin may be a formulation of an ointment, a toner, a lotion, a gel, a cream, a spray, a suspension, an emulsion, a patch, etc., but is not limited thereto. The composition according to one embodiment of the present disclosure may easily formulate the active ingredient by being carried out according to a conventional method widely known in the art, and in this case, a surfactant, an excipient, a coloring agent, a fragrance, a preservative, a stabilizer, a buffer, a suspending agent, and other commonly used auxiliary agents may be appropriately used. A dosage amount of the active ingredient of the pharmaceutical composition according to an embodiment of the present disclosure will depend on an age, sex, weight, pathologic state and severity of a subject to be administered, a route of administration, or judgment of a prescriber. Determination of an appropriate dose based on such factors is within a level of those skilled in the art, and a daily administration dose thereof may be, for example, 0.001 mg/kg/day to 10 mg/kg/day, more specifically, 0.005 mg/kg/day to 5 mg/kg/day, but is not limited thereto. In the embodiment above, the composition may be for preventing or treating skin diseases occurring due to decrease in skin wrinkles or elasticity. In an embodiment, the composition may be for preventing or treating skin diseases caused by decrease in skin barrier function. In an embodiment, the composition may be for skin antioxidation, or preventing or treating skin inflammation.

Hereinafter, the present disclosure will be described in more detail through following examples.

### [Test Example 1] In Vitro Skin Absorption Test

As an embodiment according to the present disclosure, in order to confirm an effect of pentylene glycol as an absorption promoter for riboflavin 5'-phosphate, a following experiment was conducted.

First, an aqueous solution comprising 0.05 wt% of riboflavin 5'-phosphate based on the total weight of the composition and 40 wt% of pentylene glycol, which is an absorption promoter, was prepared as an example (Example 1).

Next, as an in vitro skin permeability measurement method, after loading the composition of Example 1 prepared above in a Franz diffusion cell system (Teledyne, Chatsworth, CA, USA), a total amount of riboflavin 5'-phosphate permeated for 24 hours was measured.

Comparative examples were prepared with the same composition as the example except that Comparative Example 1 not comprising an absorption promoter was prepared, and propylene glycol (Comparative Example 2) or NEOSOLUE-AQULIO (BIS-ETHOXYDIGLYCOL CYCLOHEXANE 1,4-DICARBOXYLATE (99.97%) * TOCOPHEROL (0.03%); Franz diffusion cell system; Teledyne, Chatsworth, CA, USA) (Comparative Example 3), which is well known as an absorption promoter, was used (Table 1). For each of the comparative examples, a total amount of riboflavin 5'-phosphate permeated for 24 hours was measured using a Franz cell in the same manner as the example.

**TABLE 1**

| | Comparative Example 1 | | Comparative Example 2 | | Comparative Example 3 | | Example 1 | |
|---|---|---|---|---|---|---|---|---|
| Purified water | To 100 | | To 100 | | To 100 | | To 100 | |
| Propylene glycol | | - | | 40 | | - | | - |
| NEOSOLUE-AQULIO | | - | | - | | 40 | | - |
| Pentylene glycol | | - | | - | | - | | 40 |
| Riboflavin 5'-phosphate | | 0.05 | | 0.05 | | 0.05 | | 0.05 |

| | | | | | | | | |
|---|---|---|---|---|---|---|---|---|
| (wt%) | | | | | | | | |

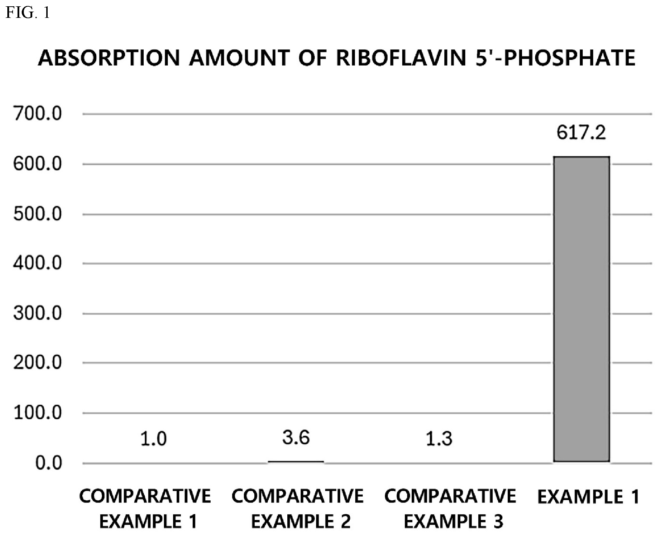

FIG. 1 is a view illustrating an absorption amount of each permeated riboflavin 5'-phosphate in Example 1 and Comparative Examples 2 and 3 by converting the absorption amount into a ratio relative to Comparative Example 1. Even though Comparative Examples 2 and 3 are organic alcohol solvents generally used in cosmetic compositions like Example 1, a skin absorption promoting effect of riboflavin 5'-phosphate appeared almost insignificantly, whereas in Example 1 comprising pentylene glycol, an absorption amount of riboflavin 5'-phosphate increased by 617 times relative to Comparative Example 1, so that it can be confirmed that a remarkably excellent skin absorption promoting effect is exhibited with respect to riboflavin 5'-phosphate.

### [Test Example 2] In Vivo Skin Absorption Test 1

As an embodiment according to the present disclosure, in order to confirm an effect of pentylene glycol as an absorption promoter for riboflavin 5'-phosphate, a following experiment was conducted.

First, an aqueous solution comprising 0.05 wt% of riboflavin 5'-phosphate based on the total weight of the composition and 40 wt% of pentylene glycol, which is an absorption promoter, was prepared as an example (Example 1).

Next, as an in vivo skin permeability measurement method, after treating 20 µl of the composition of Example 1 prepared above on a forearm part (3x3 cm) of a person having normal skin, absorption was induced for 4 minutes. After collecting 10 layers of stratum corneum using a skin stripping tape (D-Squame Stripping Disc; Cuderm Corporation, Dallas, TX, USA), riboflavin 5'-phosphate comprised in each layer of stratum corneum was quantified using Desorption Electrospray Ionization Mass Spectrometry Imaging (DESI-MSI) (Waters Milford, MA, USA).

Comparative Example 1 not comprising an absorption promoter was prepared in the above example, and Comparative Example 2 was prepared with the same composition as the example except that propylene glycol was used as an absorption promoter. For each of the comparative examples, an experiment was performed in the same manner as the example, and riboflavin 5'-phosphate comprised in each layer of stratum corneum was quantified.

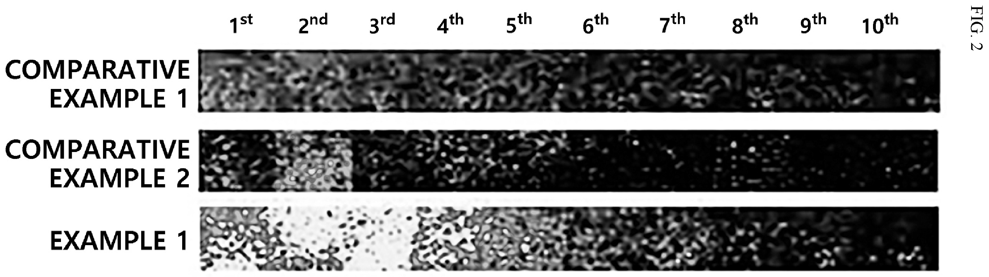

As a result, as illustrated in FIG. 2, while a skin absorption promoting effect of riboflavin 5'-phosphate appeared insignificantly in both Comparative Examples 1 and 2, an absorption amount of riboflavin 5'-phosphate remarkably increased in Example 1 comprising pentylene glycol. In addition, in Example 1, the absorption amount was highest in a third layer of the stratum corneum, and a high absorption amount was maintained even up to a fifth layer, so that it can be confirmed that a remarkably excellent skin absorption promoting effect is exhibited with respect to riboflavin 5'-phosphate.

### [Test Example 3] In Vivo Skin Absorption Test 2

As an embodiment according to the present disclosure, an effect of pentylene glycol as an absorption promoter for riboflavin 5'-phosphate was confirmed at various contents of pentylene glycol.

First, aqueous solutions comprising 0.05 wt% of riboflavin 5'-phosphate based on the total weight of the composition and 0, 10, 20, or 40 wt% of pentylene glycol, which is an absorption promoter, respectively, were prepared as examples.

**TABLE 2**

| | Comparative Example 1 | | Example 1 | | Example 2 | | Example 3 | |
|---|---|---|---|---|---|---|---|---|
| Purified water | To 100 | | To 100 | | To 100 | | To 100 | |
| Pentylene glycol | | - | | 40 | | 20 | | 10 |
| Riboflavin 5'-phosphate | | 0.05 | | 0.05 | | 0.05 | | 0.05 |

| | | | | | | | | |
|---|---|---|---|---|---|---|---|---|
| (wt%) | | | | | | | | |

Next, by experimenting in the same manner as Test Example 2 using the control group and the examples, absorption amounts of riboflavin 5'-phosphate with respect to all 10 layers of the stratum corneum were respectively quantified.

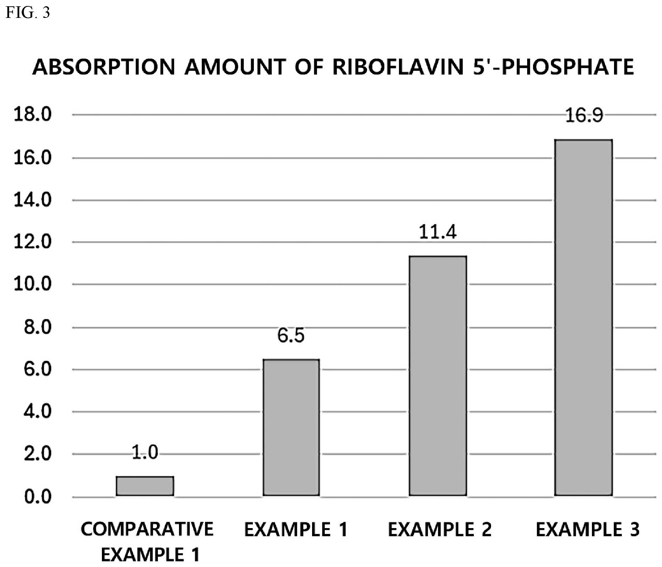

FIG. 3 is a view illustrating comparison of quantification results of a skin absorption amount of riboflavin 5'-phosphate. Relative to Comparative Example 1, it can be confirmed that when riboflavin 5'-phosphate is dissolved in pentylene glycol and absorbed, the absorption amount of riboflavin 5'-phosphate increases as a content increases.

### [Test Example 4] In Vitro Skin Absorption Test 2

As an embodiment according to the present disclosure, in order to confirm a specific effect of pentylene glycol as an absorption promoter for riboflavin 5'-phosphate, a following experiment was conducted.

First, an aqueous solution comprising 1 wt% of tocopherol or riboflavin 5'-phosphate based on the total weight of the composition and 20 wt% of pentylene glycol, which is an absorption promoter, was prepared.

**TABLE 3**

| | Comparative Example 4 | | Comparative Example 5 | | Comparative Example 6 | | Example 4 | |
|---|---|---|---|---|---|---|---|---|
| Purified water | To 100 | | To 100 | | To 100 | | To 100 | |
| Pentylene glycol | | - | | 20 | | - | | 20 |
| Tocopherol | | 1 | | 1 | | - | | - |
| Riboflavin 5'-phosphate | | - | | - | | 1 | | 1 |

| | | | | | | | | |
|---|---|---|---|---|---|---|---|---|
| (wt%) | | | | | | | | |

Next, by experimenting in the same manner as Test Example 1 using the comparative examples and the examples, tocopherol and riboflavin 5'-phosphate absorbed for 5 minutes were respectively quantified.

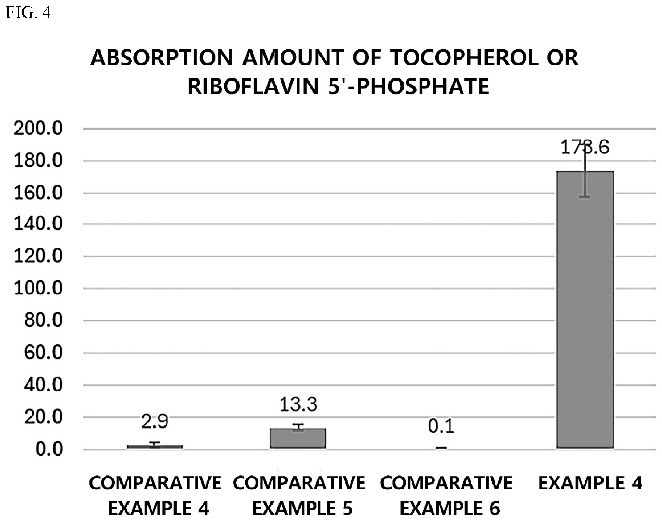

FIG. 4 is a view illustrating comparison of each quantification result of a skin absorption amount of tocopherol and riboflavin 5'-phosphate. While tocopherol has a skin absorption promoting effect of pentylene glycol (an effect of Comparative Example 5 relative to Comparative Example 4) of only about 4.6 times, it can be confirmed that riboflavin 5'-phosphate has a skin absorption promoting effect of pentylene glycol (an effect of Example 4 relative to Comparative Example 6) of about 1674.8 times, which appears very large.

The above results mean that pentylene glycol is a component that remarkably enhances absorption specifically for riboflavin 5'-phosphate, and does not remarkably promote absorption into skin for all components.

The present disclosure may provide following embodiments by way of example.

### [First Embodiment]

A composition of a skin application formulation, the composition comprising: riboflavin 5'-phosphate or a salt thereof as an active ingredient, wherein the formulation is a form in which the riboflavin 5'-phosphate or the salt thereof is dissolved in a solvent comprising pentylene glycol, and in the formulation, a degree of skin absorption of the riboflavin 5'-phosphate or the salt thereof is enhanced.

### [Second Embodiment]

The composition according to the First Embodiment, comprising the riboflavin 5'-phosphate or the salt thereof in an amount of 0.0001 to 10 wt% based on a total weight of the composition.

### [Third Embodiment]

The composition according to the First or Second Embodiment, comprising the pentylene glycol in an amount of 1 to 40 wt% based on the total weight of the composition.

### [Fourth Embodiment]

The composition according to any one of the First to Third Embodiments, wherein a weight ratio of the riboflavin 5'-phosphate or the salt thereof to pentylene glycol is 1: 1 to 10,000.

### [Fifth Embodiment]

The composition according to any one of the First to Fourth Embodiments, wherein the composition is for preventing skin wrinkle generation or improving wrinkles, or preventing skin elasticity decrease or improving skin elasticity.

### [Sixth Embodiment]

The composition according to any one of the First to Fifth Embodiments, wherein a use of the composition is one or more of skin antioxidation, skin regeneration, or prevention or improvement of skin inflammation.

### [Seventh Embodiment]

The composition according to any one of the First to Sixth Embodiments, wherein the composition is a cosmetic composition.

### [Eighth Embodiment]

The composition according to any one of the First to Seventh Embodiments, wherein the composition is a pharmaceutical composition.

## Claims

1. A composition of a skin application formulation, the composition comprising: riboflavin 5'-phosphate or a salt thereof as an active ingredient,
wherein the formulation is a form in which the riboflavin 5'-phosphate or the salt thereof is dissolved in a solvent comprising pentylene glycol, and in the formulation, a degree of skin absorption of the riboflavin 5'-phosphate or the salt thereof is enhanced.

2. The composition of claim 1, comprising the riboflavin 5'-phosphate or the salt thereof in an amount of 0.0001 to 10 wt% based on a total weight of the composition.

3. The composition of claim 1 or 2, comprising the pentylene glycol in an amount of 1 to 40 wt% based on the total weight of the composition.

4. The composition of any one of claims 1 to 3, wherein a weight ratio of the riboflavin 5'-phosphate or the salt thereof to pentylene glycol is 1: 1 to 10,000.

5. The composition of any one of claims 1 to 4, wherein the composition is a cosmetic composition.

6. The composition of any one of claims 1 to 5, wherein the composition is a pharmaceutical composition.

7. A use of the composition of any one of claims 1 to 5 for preventing skin wrinkle generation or improving wrinkles, preventing skin elasticity decrease or improving skin elasticity, skin antioxidation, skin regeneration, or preventing or improving skin inflammation.

8. A use of pentylene glycol for enhancing a degree of skin absorption of riboflavin 5'-phosphate or a salt thereof, wherein the riboflavin 5'-phosphate or the salt thereof is dissolved in a solvent comprising pentylene glycol.

9. The use of claim 8, wherein a weight ratio of the riboflavin 5'-phosphate or the salt thereof to pentylene glycol is 1: 1 to 10,000.
